**Europäisches Patentamt**

**European Patent Office**

**Office européen des brevets**

(11) Publication number : **0 544 536 A2**

# (12) EUROPEAN PATENT APPLICATION

(21) Application number : **92310854.2**

(22) Date of filing : **27.11.92**

(51) Int. Cl.$^5$ : **C12N 1/00, A01H 4/00**

(30) Priority : **28.11.91 JP 337900/91**

(43) Date of publication of application :
**02.06.93 Bulletin 93/22**

(84) Designated Contracting States :
**DE GB NL**

(71) Applicant : **Japan Tobacco Inc.**
**4-12-62 Higashishinagawa**
**Shinagawa-ku, Tokyo 140 (JP)**

(72) Inventor : **Kadotani, Naoto, c/o Japan Tobacco**
**Inc., Plant**
**Breeding and Genetics Res. Lab., 700,**
**Higashibara**
**Toyoda-cho, Iwata-gun, Shizuoka 438 (JP)**
Inventor : **Kasaoka, Keisuke, c/o Japan**
**Tobacco Inc., Plant**
**Breeding and Genetics Res. Lab., 700,**
**Higashibara**
**Toyoda-cho, Iwata-gun, Shizuoka 438 (JP)**
Inventor : **Matsushima, Hideko, c/o Japan**
**Tobacco Inc., Plant**
**Breeding and Genetics Res. Lab., 700,**
**Higashibara**
**Toyoda-cho, Iwata-gun, Shizuoka 438 (JP)**

(74) Representative : **Ruffles, Graham Keith et al**
**MARKS & CLERK 57-60 Lincoln's Inn Fields**
**London WC2A 3LS (GB)**

(54) **Method for breeding potatoes and method for producing seed potatoes.**

(57) A method for breeding potatoes by which the breeding of potatoes can be carried out in a shorter time period and smaller labor than by conventional methods is disclosed. The method for breeding potatoes according to the present invention comprises, in the order mentioned, the steps of : preparing diploid clones of potatoes ; selecting diploid clones having a desired character from said diploid clones ; preparing protoplasts from the selected diploid clones ; fusing said protoplasts to obtain tetraploid fused hybrid cells ; and growing tetraploid hybrid plants from said tetraploid fused hybrid cells.

EP 0 544 536 A2

## BACKGROUND OF THE INVENTION

I. Field of the Invention

The present invention relates to a method for breeding potatoes. The present invention also relates to a method for producing a seed potato from a virus-free seedling obtained by the method of breeding potatoes according to the present invention.

II. Description of the Related Art

In conventional methods for breeding, parents which are tetraploid plants are crossed and desired varieties are selected from segregated populations. If an egg from a mother which is to be genetically segregated in the number of "n" is crossed with a pollen from a father which is to be genetically segregated in the number of "n", the number of clones is $n^2$, so that a large number of clones that are genetically different are included in a segregated population. If "n" is 500, as many as 250,000 clones are included in the population, and it is required to select the desired clones from these clones. Thus, this selection takes a long time (not less than 10 years) and requires much labor. Further, since a new variety obtained by the conventional hybridization breeding has experienced selection tests for as long as 10 years, the possibility that the obtained new variety is infected with a virus is very high. Therefore, in Administration Center of Ministry of Agriculture, Forestry and Fishery of Japan prepares virus-free plants by apical meristem culture, and foundation stocks are derived therefrom. It takes as long as 6 years to obtain the foundation stocks from the beginning of the apical meristem culture.

To promote the efficiency of the conventional cross breeding between tetraploids (tetraploid is hereinafter also expressed as "4x"), cross breeding between a tetraploid and a diploid (diploid is hereinafter also expressed as "2x") has been developed mainly by Wisconsin University and Potato International Center (CIP) (The crossing between 4x and 2x is hereinafter also referred to as "4x X 2x"). In this method, a number of 2x clones are derived from 4x plants by using an inducer. From the thus obtained 2x clones, those having resistances to a specific disease, which have relatively good agricultural properties are selected. Thereafter, a 4x variety is crossed with the thus selected 2x clones, and good varieties are selected from the obtained clones. This method can be carried out on the condition that the 2x clones produce unreduced gamete. If the 2x clones produce unreduced gamete and have good characters, breeding can be effectively carried out by obtaining segregated populations by 4x X 2x cross breeding. However, since the number of the 2x clones that produce unreduced gametes is small, crossing between 2x clones may be necessary in order to obtain the desired 2x clones. This procedure also takes a long time.

Wenzel (1979) proposed an analytical synthetic breeding model by which heterosis is best exploited, and carried out experiments to test the model. This method comprises the steps of 1) preparing 2x clones from 4x variety, 2) preparing monoploid (hereinafter also expressed as "x") from 2x clones and then preparing homo 2x clones by diploidizing the x clones, 3) crossing a plurality of pairs of the homo 2x clones, and 4) fusing the resultants to obtain 4x clones.

Although x clones can be obtained for specific clones, they cannot be obtained for most varieties. Further, this method is complicated and takes a long time, so that it is not practical.

## SUMMARY OF THE INVENTION

Accordingly, an object of the present invention is to provide a method for breeding potatoes by which new varieties can be bred in a shorter time than that required by the conventional method. Another object of the present invention is to provide a method for producing a disease-free seed potato by which the disease-free potatoes of the variety bred by the method for breeding potatoes according to the present invention may be obtained in a shorter time than that required in the conventional method.

The present invention provides a method for breeding potatoes comprising, in the order mentioned, the steps of: preparing diploid clones of potatoes; selecting diploid clones having a desired character from said diploid clones; preparing protoplasts from the selected diploid clones; fusing said protoplasts to obtain tetraploid fused hybrid cells; and growing tetraploid hybrid plants from said tetraploid fused hybrid cells.

The present invention also provides a method for producing a seed potato comprising obtaining the seed potato from a virus-free seedling obtained by said method of the present invention.

By the method of the present invention, the desired variety can be bred in a shorter time than that required by the conventional methods, and the labor and field area required for the breeding are also smaller than those required by the conventional methods. More particularly, in the conventional cross breeding, since the desired clones are selected from about 250,000 clones, a large field area and not less than 10 years are required. In contrast, according to the method of the present invention, the same size breeding can be carried out by preparing about 1000 diploid clones, selecting about 10 good clones therefrom and breeding tetraploid varieties by cell fusion, so that the necessary field area is much smaller and the time required for the breeding can be shortened to about 6 years. Further, by the present invention, disease-free seed potato of the bred variety can also be obtained in a short time. As mentioned above, it takes about 6 years for obtaining the foundation stock by apical meristem culture, so that totally 16 years are necessary from the beginning of the breeding to the preparation of the foundation stock. By the present invention, foundation stock can be obtained in about 8 years from the beginning of the breeding.

## BRIEF DESCRIPTION OF THE DRAWINGS

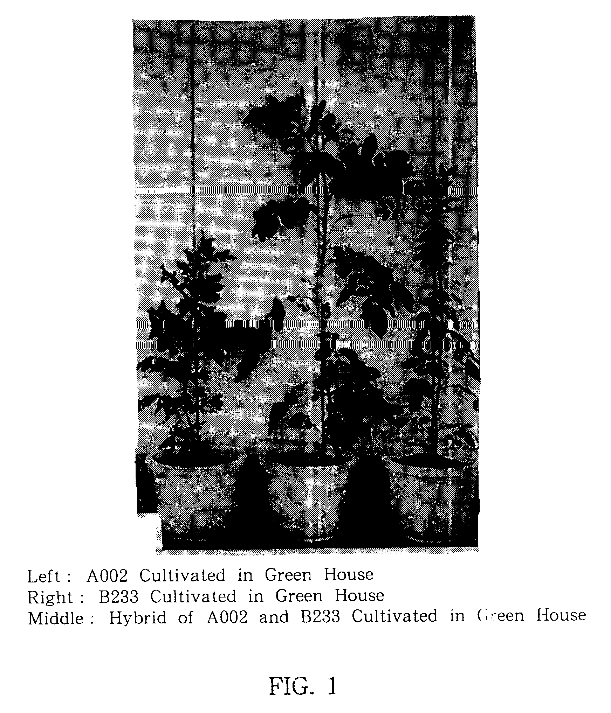

Fig. 1 is a photograph showing the 2x clones A002 and B233 subjected to the cell fusion and the 4x clone obtained by the cell fusion; and

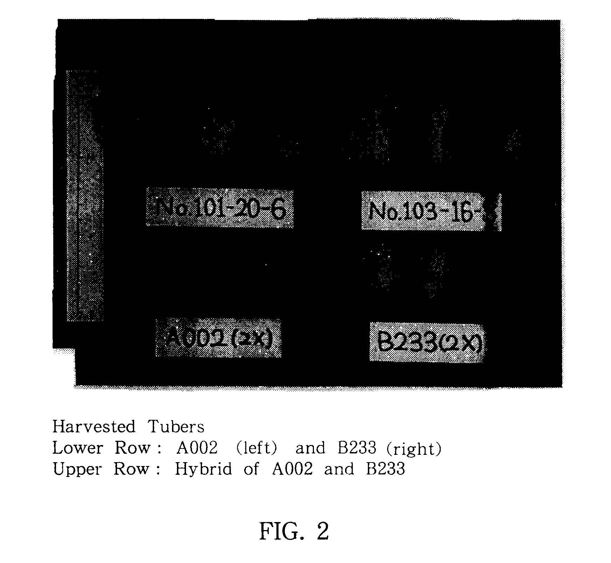

Fig. 2 is a photograph showing harvested tubers originated from different 2x clones.

## DETAILED DESCRIPTION OF THE PREFERRED EMBODIMENTS

Although not restricted, the diploid clones of potatoes may preferably be obtained by crossing an inducer with tetraploid plants. Preferred examples of the inducer include Solanum phureja IVP35 and Solanum phureja IVP48 (Hermsen, J.G. TH. and J. Verdenius 1973, Theor. Appl. Genet. 81:504-508). The diploid clones may most preferably be in the form of virus-free seedlings or microtubers derived therefrom, which are obtained by pollinating tetraploid plants with pollen of Solanum phureja and germinating the obtained diploid seeds in a tissue culture medium. As the tissue culture medium, LS medium (Linsmaier & Skoog medium) may preferably be used, although the tissue culture medium is not restricted thereto.

The diploid clones having desired characters are then selected from the thus obtained diploid clones. Although the diploid clones in the form of virus-free seedlings may be selected, it is preferred to select the clones after transplanting the diploid virus-free seedlings to a field. A necessary number of virus-free seedlings is provided for the same clones, and a part of the seedlings is transplanted to a field and tissue culture is continued for the remainder.

The transplantation may preferably be carried out by transplanting the virus-free seedlings in holes formed in the ridges in the field, and covering the ridges with a plastic film such as so called mulchfilm. The procedure of transplantation is detailed in the Example hereinbelow described.

Diploid clones having the desired characters are then selected. Examples of the desired characters include resistance to diseases for which genetical analysis have been completed, resistance to cystnematodes and resistance to PVY. Further, examples of the desired characters which can be selected when the diploid clones are transplanted in a field include starch content of tuber, chip color, shape of tuber, depth of eyes and low temperature low sugar property. The starch content means the content (wt%) of starch in the tuber (i.e., the ratio of the weight of starch in the tuber to the raw weight of the tuber). The chip color means the degree of color change to brown of the potato when the tuber is sliced into 0.5 - 1 mm thickness and the slice is fried in a vegetable oil at 180°C for 2 minutes (i.e., so called potato chip). The chip color is rated into 9 ranks. The shape of tuber is rated into 5 ranks according to the goodness of the outer appearance of the tuber. The depth of eyes means the depth of the concaves around germs (the concave is called eye), and the depth is rated into 5 ranks by gross observation. The low temperature low sugar property means the chip color after storing the potato at 6°C for 3 weeks. The lower the degree of color change to brown, the better the low temperature low sugar property.

By the above-described selection, at least two good clones are selected and protoplasts of the selected clones are prepared according to a conventional method. The protoplasts may preferably be prepared from the corresponding virus-free seedlings which are continuously tissue-cultured during the selection in the field is carried out.

The thus obtained protoplasts are then fused. The fusion can be carried out according to a conventional method. The fusion may preferably be carried out by the electrofusion method which per se is well-known in the art. Two of the selected clones are fused. It is preferred that two clones originated from different varieties be fused since the heterosis can be exploited, so that the possibility of obtaining a good variety is promoted.

Then tetraploid fused hybrid cells are selected and cultured. In a preferred embodiment, the protoplasts of one of the clones are mesophyll protoplasts which cannot divide unless they are fused with a cell having an ability to undergo cell division, and the protoplasts of the other clones are chlorotic mesophyll protoplasts originating from a plant treated with norflurazon. The mesophyll protoplasts which cannot divide by itself can be obtained by treating the mesophyll protoplasts with iodoacetamide (IOA). After the fusion, the cell suspension is centrifuged and the cells in the fraction precipitated by the centrifugation is cultured. By this procedure, the tetraploid fused hybrid cells can be selected. This procedure is detailed in the Example hereinbelow described and also described in co-pending U.S. patent application serial No. 07/897,101 and in co-pending European patent application No. 92 109 722.6.

From the thus obtained tetraploid fused hybrid cells, whole plants are regenerated according to a conventional method. The protoplast-culturing medium employed in this step may be, for example, P-1 medium, the callus-growing medium may be, for example, C-1 medium and the regeneration medium may be, for example, S-1 medium, although the media which may be employed are not restricted thereto.

The regenerated plants may be obtained as virus-free seedlings. The number of the seedlings may be increased as necessary, and a part of the seedlings may be transplanted to a field and the remainder may be tissue-cultured. The tubers are then harvested and the clones having the desired characters may be selected again. The transplantation may be carried out by the preferred method described above.

Seed potatoes may be obtained from the thus finally selected clone by growing the virus-free seedlings of the clone which are continuously tissue-cultured during the selection in the field is carried out, transplanting the virus-free seedlings to a field and by cultivating the transplanted plants. Alternatively, microtubers are derived from the virus-free seedlings by the method well-known in the art, and the microtubers may be transplanted to a field and cultivated. Thus, from the virus-free seedlings obtained by the method of the present invention, disease-free seed potatoes may be obtained in much shorter time than in the conventional method.

The method for breeding potatoes according to the present invention has the following advantages: That is, diploid clones are prepared and the selection is made on the diploid clones. Since the goodness of the characters is much more distinct in diploid clones than in tetraploid clones, it is assured that clones having good characters be selected by selecting only a small number of clones (e.g., up to 10 clones) having the desired characters. Further, the number of segregated diploid clones is much smaller than the segregated tetraploid clones. As mentioned above, if the number of segregated tetraploid clones is about 250,000, the number of diploid segregated clones is about 1000. Thus, the labor required for the selection is much reduced. Further, the above-mentioned preferred embodiment of the method of the present invention has an advantage that virus-free seedlings corresponding to the clones selected in the field exist. In this method, since protoplasts to be subjected to the cell fusion can be obtained from the virus-free seedlings, the time and labor for obtaining the virus-free seedling from the plant selected in the field are omitted. Further, since the tetraploid hybrid plants are obtained by cell fusion, the advantages of the cross breeding can be obtained. Especially, when two protoplasts originating from different varieties are fused, the advantage of heterosis can be obtained. Still further, after the final selection of the tetraploid hybrid plants, disease-free seed potatoes can be obtained by growing the virus-free seedlings corresponding to the selected clones, transplanting the seedlings to the field and cultivating the transplanted plants. Thus, the time required for producing the disease-free seed potatoes can be shortened.

The invention will now be described by way of examples thereof. It should be noted that the examples are presented for the illustration purpose only and should not be interpreted in any restrictive way.

Example 1

(A) Varieties Employed for Breeding

Ten varieties of potato, that is, May Queen, Konafubuki, Danshaku, Norin 1, Toyoshiro, Eniwa, Shimakei 537, Hokkai 65, Atlantic and Hokkaikogane were employed.

(B) Preparation of Diploid Clones

1) The plants of the above-mentioned ten varieties were cultivated in a green house at 20°C during daytime and at 15°C during nighttime in 24 cm pots. When the plants flowered, the plants were pollinated with pollen of Solanum phureja IVP-35 or IVP-48 (inducer) so as to induce parthenogenesis. From the obtained seeds, those having no germinal spots were selected by gross observation to obtain 2x seeds.

2) The obtained seeds were treated with 30 ppm of gibberellin solution for 30 minutes to halt the dormancy and the resulting seeds were immersed in 70% ethanol for several seconds and in 1% sodium hypochloride for 5 minutes to sterilize the surfaces of the seeds. The seeds were then well washed with sterilized water and aseptically sown on LS agar medium. The seeds were cultivated in an incubator at 20°C, under illumination of 5000 lux for 16 hours a day (the incubator employing these conditions is hereinafter referred to as "20°C incubator" for short).

3) Among the germinated virus-free plants, those having violet bands at nodes were removed because they had been crossed (i.e., not the product of parthenogenesis). Those which did not have such violet bands were tested for the number of chromosomes using a cell sorter. Thereafter, 1046 clones which were confirmed to be 2x were cultured in test tubes and maintained.

(C) Selection Based on Specific Characters at 2x Level

1) Growing of Cultured Seedlings

In plant boxes, 50 ml/box of LS liquid medium was placed and 5 - 10 nodes/box of 2x virus-free plants were placed, and the plants were cultured in the 20°C incubator for 3 weeks, thereby extending stems and leaves from the nodes. Then the extended stem and leaf portions were cut into 2 - 3 cm length and the resulting stems with leaves were transplanted to LS agar medium in plant boxes, so as to root the plants and to obtain seedlings. The seedlings obtained by this method are strong enough to be transplanted to the field.

2) Transplantation to Field and Cultivation

When the soil contained suitable moisture, ridges with a height of 20 cm were formed with intervals of one meter. The ridges were covered with silver-colored mulchfilm. Totally 200 kg of a mixed fertilizer (6-11-7) was applied to the ridges. In cases where the virus-free seedlings are directly transplanted to the field, holes having a diameter of about 10 cm were formed in the mulchfilm and planting holes having a diameter of about 10 cm and a depth of about 10 cm were formed under the holes formed in the mulchfilm. Each virus-free seedling was planted in each hole. After the transplantation, the ridges were covered with transparent mulchfilm to keep the moisture in the soil and temperature of the soil appropriately. When the seedlings grew so that they touch the mulchfilm, the film was cut and then the plants were cultivated according to the conventional potato mulchfilm cultivation. In cases where the seedlings are grown in a green house, seedlings with a height of about 10 cm were transplanted to a field according to a conventional method and cultivated. When the stems and leaves were wilting, tubers were harvested and selection was made according to the characteristics of the harvested tubers.

3) Method of Selection

The time required for the breeding can be shortened if the selection of the desired clones can be made for the first generation (the plants obtained by transplanting the virus-free seedlings as described in the preceding section). Thus, the correlations among the characteristics of the first generation, the second generation derived from the tubers of the first generation and of the third generation derived from the tubers of the second generation were examined, which are shown in Table 1.

Table 1    Correlations Between Phenotypes of Each Generation

| Characters | Correlation Coefficient | | |
|---|---|---|---|
| | 1st – 3rd Generations | 2nd – 3rd Generations | 1st – 2nd Generations |
| Height of Plant | 0.588** | 0.739** | 0.764** |
| Weight of Tubers per Plant | 0.370* | 0.502** | 0.661** |
| Number of Tubers per Plant | 0.466** | 0.573** | 0.637** |
| Weight of Upper Grade Tubers*** | 0.297 | 0.517** | 0.530** |
| Shape of Tubers | 0.538** | 0.629** | 0.639** |
| Starch Content | 0.865** | 0.920** | 0.907** |
| Chip Color (20°C, 9 weeks) | 0.833** | 0.899** | 0.856** |
| Chip Color (6 °C, 2 weeks) | 0.631** | 0.726** | 0.626** |

*: significant at 5%

**: significant at 1%

***: selected 10 tubers according to their shape and weight which is medium (between about 10 – 100 g)

As shown in Table 1, the correlations among the first, second and the third generations are very high for the starch content and chip color. Thus, the selection of the clones having good starch and good chip color can be carried out in the first generation.

Therefore, in the first generation, those exhibited abnormal growth and those produced tubers whose outer appearances were extremely bad were removed, and the selection was made based on the starch content and the chip color. The selection of the second generation was made based on the shape of the tubers and the depth of the eyes.

4) Results of Selection of Diploid Clones

The 1046 diploid clones obtained as described above were subjected to the selection mentioned above, and 4 clones originating from May Queen, 3 clones originating from Konafubuki, 1 clone originating from Danshaku and 3 clones originating from Atlantic were selected as good diploid clones.

(D) Fusion of Selected Diploid Clones and Preparation of Tetraploid Hybrid Plants

1) Protoplasts were prepared from the aseptically cultured clones which were the same as the selected good diploid clones originating from May Queen and Konafubuki, and the protoplasts originating from different varieties were fused according to the method described below. To effectively select the tetraploid fused hybrid cells alone, the method in which the chlorotic mesophyll protoplasts and IOA-treated mesophyll protoplasts mentioned above was employed. The method is hereinbelow described.

2) Preparation of Chlorotic mesophyll plants

To each plant box (300 ml, commercially available from Veldy) containing 20 ml of LSP5 liquid medium having the composition shown in Table 2, three single nodes of a virus-free seadling of the diploid clone grown in a test tube containing LS agar medium, which was originated from Konafubuki, were placed and cultured in the 20°C incubator for 2 weeks. When the plants were grown to 5 - 7-leaf stage, norflurazon was added to a final concentration of 20 ppm. The plants were cultured for another 10 days and the leaves at the upper portions of the plants turned chlorotic, so that plants suitable for the separation of chlorotic mesophyll protoplasts were obtained.

### Table 2 Composition of LSP5 Medium

| Component | Concentration (g/l) |
|---|---|
| LS Medium* | |
| myo-Inositol | 0.1 |
| Thiamine HCl | 0.001 |
| Sucrose | 5.0 |
| (pH 5.6) | |

*: Linsmaier and Skoog medium (1965)

3) Isolation of Protoplasts

The chlorotic mesophyll of the thus obtained chlorotic plants was cut into pieces with a width of 1 - 2 mm, and the pieces were treated with isolation enzyme solution POT-9 having the composition shown in Table 3 at 25°C for 16 - 18 hours so as to isolated protoplasts. After the enzyme treatment, the protoplasts were washed three times with 0.5 M mannitol solution.

## Table 3    Composition of Enzyme Solution POT-9

| Component | Concentration (g/l) |
|---|---|
| Meicelase (P-1)[*] | 5.0 |
| Pectriase (Y-23)[**] | 0.5 |
| 2-(N-Morpholino)ethanesulfonic Acid (MES) | 1.0 |
| Polyvinylpyrrolidone (PVP 40) | 20.0 |
| Mannitol | 91.0 |
| (pH 5.6) | |

*: commercially available from Meiji Seika Kaisha, Ltd.

**: commercially available from Seishin Pharmaceutical Co., Ltd.

Green protoplasts were obtained by treating the mesophyll of the virus-free seedlings of the diploid clones originating form May Queen, which were grown on LS agar medium in test tubes, in the same manner as described for the chlorotic mesophyll protoplasts.

4) Treatment of Green Mesophyll Protoplasts with IOA

The obtained green mesophyll protoplasts were treated with 10 mM IOA at 4°C for 15 minutes and the resultant was washed three times with 0.5 M mannitol solution.

5) Cell Fusion

A suspension of the chlorotic mesophyll protoplasts having a population density of $10^4$ - $10^5$ protoplasts/ml was mixed with an equivolume of a suspension of the IOA-treated green mesophyll protoplasts having the same population density. The mixture was then subjected to electrofusion using an electrofusion apparatus (SSH-1, commercially available from Shimazu Corp., Kyoto, Japan). To increase the rate of fusion, the electrofusion was performed after suspending the protoplasts in 0.45 M mannitol solution containing 1 mM calcium chloride solution. The conditions of the electrofusion are shown in Table 4.

| Table 4 Conditions of Electrofusion | |
|---|---|
| Frequency of High Frequency Wave | 1 MHz |
| Voltage of High Frequency Wave | 100 V/cm |
| Time for Applying Voltage | 10 - 20 sec. |
| Pulse (rectangular wave) Voltage | 1.5 KV/cm |
| Pulse Width | 15 μsec. |
| Interval Between Pulses | 0.2 - 1.0 sec. |
| Number of Pulses Applied | 2 - 10 times |

6) Selection of Tetraploid Fused Hybrid Cells

After the electrofusion, the resulting cell suspension was centrifuged at 250 rpm for 5 - 10 minutes using a low speed centrifuge and the supernatant was removed. To the precipitate, 0.45 M mannitol solution was added to suspend the cells and the suspension was centrifuged in the same conditions as just mentioned above, followed by removal of the supernatant. This operation was repeated totally three times.

As a result of this operation, most of the chlorotic protoplasts having smaller specific gravities were removed together with the supernatant, and only the protoplasts between the green mesophyll protoplasts having larger specific gravities and the fused cells between green protoplasts and chlorotic protoplasts were collected.

7) Regeneration of Plants from Tetraploid Fused Hybrid Cells

As the medium for culturing the protoplasts, P-1 medium having the composition shown in Table 5 was employed.

## Table 5    Composition of Medium for Culturing Protoplasts (P-1 Medium)

| Component | Concentration (mg/l) |
|---|---|
| MS Medium[*] from Which $NH_4NO_3$ is Removed | |
| myo-Inositol | 1,000.0 |
| 2,4-Dichlorophenoxyacetic Acid (2,4-D) | 0.1 |
| Naphthaleneacetic Acid (NAA) | 0.5 |
| Benzylaminopurine (BA) | 0.5 |
| Glucose | 5,000.0 |
| Sucrose | 10,000.0 |
| Mannitol | 46,000.0 |
| (pH 5.6) | |

[*]: Murashige and Skoog Medium (1962)

The cells collected by the above-described centrifugation were suspended in P-1 medium to a final concentration of $10^4$ - $10^5$ cells/ml. On the other hand, P-1 medium further containing 1.6 wt% sea plaque agarose kept at 40°C was prepared and mixed with equivolume of the above-described cell suspension in P-1 medium. Before the medium solidified, 10 droplets/dish of the mixture having a volume of 100 μl/droplet were placed in each petri dish having a diameter of 6 cm (commercially available from Corning Glass) . After the droplets completely solidified, 3 ml of P-1 liquid medium was placed around each droplets, and the cells were cultured at 25°C in the dark. As mentioned above, since the green mesophyll protoplasts were treated with IOA and so they cannot divide unless they are fused with the chlorotic protoplasts, only the desired tetraploid fused hybrid cells can undergo cell division.

After 20 - 40 days' culture, the liquid medium around the droplets was removed and the droplets were crushed to disperse the divided cells. To the cells, C-1 medium for growing callus having the composition shown in Table 6 was added and the cells were cultured in an incubator at 25°C under illumination of 5000 lux for 24 hours a day.

Table 6    Composition of Colony Growth Medium (C-1
             Medium)

| Component | Concentration (mg/l) |
|---|---|
| MS Medium* from Which $NH_4NO_3$ is Removed | |
| $NH_4NO_3$ | 300.0 |
| Naphthaleneacetic Acid (NAA) | 0.1 |
| Benzylaminopurine (BA) | 0.5 |
| Sucrose | 3,000.0 |
| Mannitol | 50,000.0 |
| (pH 5.6) | |

*: Murashige and Skoog Medium (1962)

The calli grown to 1 - 3 mm in 10 - 20 days were transplanted to S-1 medium for regeneration having the composition shown in Table 7, and were cultured in the 20°C incubator.

Table 7    Regeneration Medium (S-1 Medium)

| Component | Concentration (mg/l) |
|---|---|
| MS Medium[*] from Which $NH_4NO_3$ is Removed | |
| $NH_4NO_3$ | 300.0 |
| Indoleacetic Acid (IAA) | 0.3 |
| Zeatin | 2.0 |
| Coconut Water[**] | 2.0(%) |
| Sucrose | 3,000.0 |
| Mannitol | 35,000.0 |
| Gellan Gum (Gelrite) | 2,000.0 |
| (pH 5.8) | |

[*]: Murashige and Skoog Medium (1962)

[**]: commercially available from GIBCO

As a result, as shown in Table 8, 704 plants were generated from the tetraploid fused hybrid cells prepared by cell fusion of 6 types of pairs. The thus obtained tetraploid hybrid plants were transplanted to LS agar medium contained in test tubes, and grown after rooted.

Table 8 Regenerated Plants and Hybrid Plants Produced by Combinations of Cells Fused

| Combination of Cells Fused* | Number of Regenerated Plants | Results of Tests Confirming Formation of Hybrid Plants | |
|---|---|---|---|
| | | Number of Plants Examined | Number of Hybrid Plants** |
| A002 + B233 | 489 | 295 | 254 |
| A017 + B233 | 69 | 3 | 2 |
| A022 + B233 | 48 | 3 | 2 |
| A073 + B233 | 39 | 4 | 3 |
| A118 + B233 | 40 | 0 | 0 |
| A136 + B233 | 18 | 0 | 0 |
| Total | 703 | 305 | 261 |

*: "A" indicates the diploid clones of May Queen and "B" indicates the diploid clones of Konafubuki.

**: Number of plants which were confirmed to be hybrids by RFLP (restriction fragment length polymorphism) and by morphological examination

8) Confirmation of Tetraploid

Tetraploid plants which were thought to be hybrid plants were selected by examining the morphology of the regenerated plants and by checking the number of chromosomes by a cell sorter. Nuclear DNAs were extracted from the selected plants and the evaluation whether the plants were hybrids or not was made by RFLP using a DNA probe originating from glutamine synthetase gene.

The tetraploid plants were maintained by subculturing the plants on LS agar medium in test tubes.

(E) Final Selection of Bred Variety

1) The cultured seedlings of the tetraploid hybrid plants obtained in (D) were cultivated in pots in a green house at 20°C during daytime and at 15°C during nighttime to obtain minitubers. On the other hand, the cultured seedlings were transplanted to the field in the same manner as in (C)2) to obtain small tubers. Heterosis was observed for the plant morphology and tubers of the tetraploid hybrid plants.

2) The minitubers and small tubers obtained as described above were cultivated in the field. For the tetraploid plants of 53 clones, date of germination, date of flowering, color of flower, morphology of the plant portion above the soil, aging, height of plant, weight of tuber, number of tuber, starch content, existence of secondary growth, shape of tuber, depth of eyes, outer appearance of tuber, color of flesh, and chip color were examined. As a result, two clones were selected as good clones. The selected good clones were No. 101-20-6 and 103-16-3.

3) The thus selected good clones were cultivated according to a conventional method and the characters of the good clones were compared with those of the varieties now cultivated. The results are shown in table 9.

Table 9   Characters of Portions above the Ground and Tubers

| Variety | Height of Plant | Yield/10 a | Starch Content | Chip Color 1* | Chip Color 2** |
|---|---|---|---|---|---|
| No. 101-20-6 | 36.7 cm | 5.6 t | 15.3% | ◎ | △ |
| No. 103-16-3 | 35.5 cm | 5.5 t | 15.5% | ◎ | △ |
| May Queen | 38.8 cm | 4.8 t | 12.7% | ○ | × |
| Danshaku | 34.4 cm | 5.5 t | 13.4% | △ | × |
| Toyoshiro | – | – | 13.6% | ◎ | ○ |
| Norin 1 | – | – | 14.6% | × | × |
| Konafubuki | 48.0 cm | – | 18.1% | ○ | × |

*: Tubers tested were those stored at room temperature after harvest.

**: Tubers tested were those stored at low temperature after harvest (6 °C, 2 weeks)

Symbols Indicating Chip Colors:

◎: very good,

○: good

△: little poor

×: poor

As can be seen from Table 9, the yields of the good clones were comparative to those of May Queen and Danshaku, the starch contents were somewhat higher than that of Toyoshiro and the chip colors were comparative to that of Toyoshiro.

(F) Production of Disease-free Seed Potato

From the virus-free seedlings of the good clones maintained by the culture in (D), minitubers were produced in a green house and the minitubers were transplanted to the field and cultivated to obtain disease-free seed potatoes.

Although the invention was described by way of preferred embodiments thereof, it is apparent for those skilled in the art that various modifications may be made without departing from the spirit and scope of the present invention.

**Claims**

1. A method for breeding potatoes comprising, in the order mentioned, the steps of:
   preparing diploid clones of potatoes;
   selecting diploid clones having a desired character from said diploid clones;
   preparing protoplasts from the selected diploid clones;
   fusing said protoplasts to obtain tetraploid fused hybrid cells; and
   growing tetraploid hybrid plants from said tetraploid fused hybrid cells.

2. The method of claim 1, wherein said diploid clones are prepared by crossing an inducer with tetraploid plants.

3. The method of claim 1, wherein said diploid clones are in the form of cultured virus-free seedlings.

4. The method of claim 1, wherein said step of selecting diploid clones having a desired character is carried out on the diploid clones transplanted to a field.

5. The method of claim 1, wherein said character is starch content, chip color, shape of tuber, depth of eye, and/or low temperature low sugar property.

6. The method of claim 1, wherein said fused hybrid cells are prepared by fusing protoplasts originated from diploid clones of different varieties.

7. A method for producing a seed potato comprising obtaining the seed potato from a bacterium-free seedling obtained by the method of claim 1.

Left : A002 Cultivated in Green House
Right : B233 Cultivated in Green House
Middle : Hybrid of A002 and B233 Cultivated in Green House

FIG. 1

Harvested Tubers
Lower Row : A002 (left) and B233 (right)
Upper Row : Hybrid of A002 and B233

FIG. 2